# EUROPEAN PATENT SPECIFICATION

(11) **EP 1 674 449 B1**
(45) Date of publication and mention of the grant of the patent: **16.12.2009**
(21) Application number: 05027959.5
(22) Date of filing: 20.12.2005
(51) Int. Cl.: C07C 249/04, C07C 251/44

(54) **Process for producing cyclohexanone oxime**
Verfahren zur Herstellung von Cyclohexanonoxim
Procédé de préparation de l'oxime de la cyclohexanone

(30) Priority: 22.12.2004 JP 2004370727
(43) Date of publication of application: 28.06.2006
(73) Proprietor: Sumitomo Chemical Company, Limited, Tokyo 104-8260 (JP)
(72) Inventor: Trivellone, Franco, 20052 Monza (IT); Furlan, Piero, 31100 Treviso (IT); Oikawa, Miyuki, Niihama-shi Ehime-ken (JP)
(74) Representative: Vossius & Partner

(56) References cited:
- EP-A- 0 496 385
- EP-A- 1 433 777

## Description

The present invention relates to a process for producing cyclohexanone oxime by an ammoximation reaction of cyclohexanone. Cyclohexanone oxime is useful, for example, as a raw material for ε-caprolactam.

As one of processes for producing cyclohexanone oxime, a process of carrying out the ammoximation reaction of cyclohexanone with hydrogen peroxide and ammonia in the presence of a titanosilicate catalyst has been proposed (for example, refer to the patent literatures 1 to 4 indicated below). The process does not require neutralization of sulfuric acid with ammonia which neutralizing is required in a conventional process for carrying out oximation with hydroxyl ammine sulfate, and it also has an advantage that separation of a product from the catalyst is easy because it is a solid catalyst reaction process.

The above-mentioned ammoximation reaction process is advantageous when it is carried out in a continuous system from the viewpoints of productivity and operation of the process. For example, it is disclosed in the patent literatures 2 and 3 that the ammoximation reaction is carried out in a continuous system by separating through a filter a liquid phase of a reaction mixture in a reactor in which mixture a catalyst is dispersed while feeding cyclohexanone, hydrogen peroxide and ammonia into the reactor. Further, it is disclosed in the patent literature 4 that the ammoximation reaction is carried out in a continuous system while feeding a catalyst together with raw materials into a reactor.

[Patent literature 1] JP-A-62-59256 (1987)
[Patent literature 2] JP-A-6-49015 (1994)
[Patent literature 3] JP-A-6-92922 (1994)
[Patent literature 4] JP-A-2000-72737

It is the purpose of the present invention to provide a process which is able to produce cyclohexanone oxime in a higher yield by further improving the selectivity of cyclohexanone oxime in the above-mentioned continuous ammoximation reaction process.

The present invention provides a process for producing cyclohexanone oxime in a continuous reaction system by preparing a reaction mixture in a reactor in which mixture a titanosilicate catalyst is dispersed, and feeding cyclohexanone, hydrogen peroxide and ammonia into the reactor through a feeding orifice of cyclohexanone, a feeding orifice of hydrogen peroxide and a feeding orifice of ammonia, respectively, wherein the above mentioned reaction mixture forms a circulating flow in the reactor, and the feeding orifice of hydrogen peroxide and the feeding orifice of ammonia are provided in the above mentioned order on the downstream side of the feeding orifice of cyclohexanone along said circulating flow.

That is, upon producing cyclohexanone oxime, the circulating flow of the reaction mixture is formed in the reactor which contains the mixture having the catalyst dispersed therein, and it is characterized in that along a flowing direction of the circulating flow in the reactor from its upstream side toward its downstream side, cyclohexanone is supplied to the reactor, hydrogen peroxide is supplied to the reactor on its downstream side and ammonia is supplied to the reactor on a its more downstream side as in thus mentioned order. As a result, cyclohexanone is supplied most upstream, ammonia is supplied most downstream and hydrogen peroxide is supplied between them.

The present invention further provides a reaction apparatus in which the above mentioned process is carried out for producing cylohexyanone oxime as described above, that is, a reaction apparatus wherein the circulating flow is formed, and the feeding orifice of hydrogen peroxide and the feeding orifice ammonia are located in the thus listed order on the downstream side of the feeding orifice of cyclohexyanone along the flowing direction of the circulating flow. In other words, in the reaction apparatus according to the present invention, the feeding orifice of cyclohexyanone is located upstream, the feeding orifice ammonia is located downstream and the feeding orifice of hydrogen peroxide is located between the other two feeding orifices.

According to the present invention, the continuous ammoximation reaction of cyclohexanone can be carried out in a high selectivity and cyclohexanone oxime can be produced in a high yield.
Fig. 1 schematically shows a cross sectional view of an example of a reaction apparatus which is used for the process of the present invention.
Fig. 2 schematically shows a perpendicular cross sectional view of another example of a reaction apparatus which is used for the process of the present invention.
In the drawings, the numerals indicate the following members, respectively:
- 1: ... reactor
- 2: ... feeding pipe of cyclohexanone
- 3: ... feeding pipe of hydrogen peroxide
- 4: ... feeding pipe of ammonia
- 1a: ... discharge orifice of reaction mixture
- 2a: ... feeding orifice of cyclohexanone
- 3a: ... feeding orifice of hydrogen peroxide
- 4a: ... feeding orifice of ammonia
- 1': ... reaction mixture
- 2': ... cyclohexanone
- 3': ... hydrogen peroxide
- 4': ... ammonia
- 5: ... bulkhead
- 6: ... mixing impeller

The present invention is specifically described below. The titanosilicate as the catalyst used in the present invention is preferably a zeolite which contains titanium, silicon and oxygen as elements forming its framework. The framework may substantially be composed of titanium, silicon and oxygen, or the framework may further contain other additional element(s). As the titanosilicate catalyst, those having an atomic ratio of silicon/titanium in the range between 10 and 1000 are preferably used, and the catalyst may be in the form of, for example, fine powder or pellets. The titanosilicate can be prepared by a process described in, for example, JP-A-56-96720 (1981).

Cyclohexanone oxime can be produced by carrying out the ammoximation reaction of cyclohexanone with hydrogen peroxide and ammonia in the presence of a catalyst while using the above-mentioned titanosilicate as the catalyst.

Cyclohexanone as the raw material may be obtained, for example, by the oxidation reaction of cyclohexane, by the hydration and dehygrogenation reactions of cyclohexene, or by the hydrogenation reaction of phenol.

Hydrogen peroxide is usually produced by a so-called anthraquinone method. Hydrogen peroxide is usually commercially available as an aqueous solution with a concentration of 10 to 70% by weight of hydrogen peroxide, and such a solution can be used. Further, hydrogen peroxide can also be produced by reacting hydrogen with oxygen in an organic solvent in the presence of a solid catalyst having metal palladium supported thereon. When hydrogen peroxide according to such a process is used, an organic solvent solution of hydrogen peroxide which solution is obtained by separating the catalyst from the reaction mixture may be used in place of the above mentioned hydrogen peroxide aqueous solution. The amount of hydrogen peroxide used is usually in the range between 0.5 mol and 3 mol and preferably in the range between 0.5 mol and 1.5 mol based on one mole of cyclohexanone. It is noted that a stabilizer such as a phosphate (such as sodium phosphate), a polyphosphate (such as sodium pyrophosphate and sodium tripolyphosphate), pyrophosphoric acid, ascorbic acid, ethylenediaminetetraacetic acid, nitrotriacetic acid, aminotriacetic acid, and diethylenetriaminepentaacetic acid may be added to hydrogen peroxide.

Ammonia may be used in a gaseous form or a liquid form, and ammonia may be used as a solution in water or in an organic solvent. The amount of ammonia used is preferably 1 mol or more and preferably 1.5 mol or more per one mole of cyclohexanone.

The above mentioned ammoximation reaction may be carried out in a solvent, and as such a reaction solvent, an alcohol (such as methyl alcohol, ethyl alcohol, n-propyl alcohol, isopropyl alcohol, n-butyl alcohol, sec-butyl alcohol, tert-butyl alcohol and tert-amyl alcohol), water, or any mixture thereof is preferably used.

The reaction temperature of the above-mentioned ammoximation reaction is usually in the range between 50°C and 120°C and preferably in the range between 70°C and 100°C. Further, the reaction pressure may be normal pressure, but the reaction is preferably carried out under pressure for increasing the amount of ammonia dissolved in the liquid phase of the reaction mixture. When carrying out the reaction under pressure, the pressure may be adjusted using an inert gas such as nitrogen or helium.

In the present invention, the above mentioned ammoximation reaction is carried out in a continuous system. Such a continuous reaction is usually carried out by retaining a predetermined amount of the reaction mixture in which the catalyst is dispersed in a continuous reactor such as a stirred vessel type reactor or a loop type reactor, and feeding cyclohexanone, hydrogen peroxide, ammonia and, if necessary, a solvent and the like while discharging an amount of the reaction mixture which amount is generally equal to the total amount of those raw materials supplied into the reactor. The discharge of the reaction mixture herein is preferably carried out by taking out only its liquid phase through a filter and the like while retaining the catalyst in the reactor. When the catalyst is also discharged together with the liquid phase, an amount of the catalyst also has to be supplied into the reactor. As the catalyst which is to be supplied, a new catalyst which has not been used for the reaction can be used, but the recovered catalyst which has been separated from the discharged reaction mixture can be used again after subjecting it to regeneration processing if necessary, for example, a treatment of removing organic materials by calcination, and the new catalyst and the recovered catalyst may be used in combination.

The concentration of the catalyst in the reaction mixture depends on the activity of the catalyst, reaction conditions and the like, and it is usually in the range between 1 g/L and 100 g/L, preferably in the range between 5 g/L and 100 g/L, and more preferably in the range between 10 g/L and 100 g/L as a weight of the catalyst per unit volume of the reaction mixture (catalyst + liquid phase). It is noted that, upon the initiation of the reaction, for example, a catalyst dispersion in which the catalyst is dispersed in a solvent is charged in the reactor, and feeding of the above mentioned raw materials is started as described above, while discharging of the reaction mixture is started. As the reactor, a glass lined reactor or a reactor made of a stainless steel is preferably used from the viewpoint of preventing decomposition of hydrogen peroxide.

According to the present invention, in the above mentioned continuous ammoximation reaction process, the circulating flow of the reaction mixture is formed in the reactor in which mixture the catalyst is dispersed, and cyclohexanone, hydrogen peroxide and ammonia are fed thereinto through the feeding orifice of cyclohexanone, the feeding orifice of hydrogen peroxide and the feeding orifice of ammonia, respectively. Upon such feeding, the positional relationship of the respective feed orifices is designed so that the feeding orifice of hydrogen peroxide and the feeding orifice of ammonia are arranged in the above mentioned order along a flowing direction of the above mentioned circulating flow on the downstream side of the feeding orifice of cyclohexanone. As a result, a side reaction such as a condensation reaction of cyclohexanone can be suppressed by forming the circulating flow of the reaction mixture in which the catalyst was dispersed in the reactor and feeding the respective raw materials into the reactor under the predetermined relationship as to the feeding positions of the raw materials, so that the selectivity of cyclohexanone oxime can be improved.

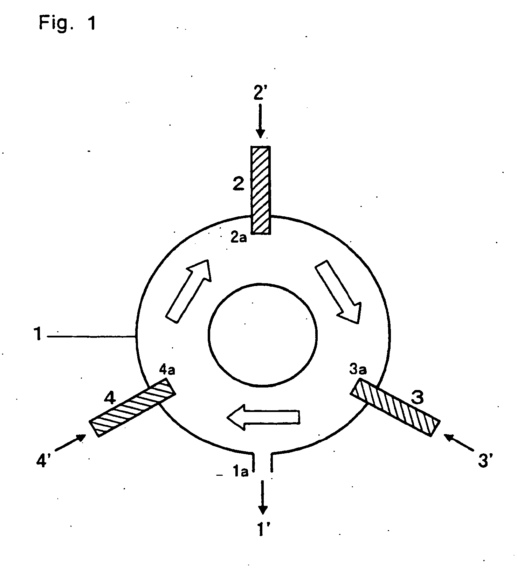

Fig. 1 schematically shows a cross sectional view of an example of the reaction apparatus useful in the continuous ammoximation reaction process according to the present invention. Namely, the circulating flow of the reaction mixture indicated by the white arrow marks is formed by a pump and the like in a loop-type (or a doughnut-type) reactor 1. Cyclohexanone 2' is fed into the circulating flow through the feeding orifice 2a at an end portion of the feed pipe 2, hydrogen peroxide 3' is fed on the downstream side of the feeding orifice 2a through the feeding orifice 3a at the end portion of the feeding pipe 3, and ammonia 4' is fed on the downstream side of the feeding orifice 3a through the feeding orifice 4a at the end portion of the feeding pipe 4. Further, the reaction mixture 1' an amount of which is nearly the same as a total amount of these fed materials is discharged through a discharge orifice 1a which is provided at the reactor 1. It is noted that the meaning of "downstream" (and also the meaning of "upstream") as to the positional relationship of the feeding orifices 2a, 3a and 4a is used in a relative meaning. Thus, for example, when the feeding orifice 3a of hydrogen peroxide is set as a basis, it can be considered that the feeding orifice 4a of ammonia is arranged on the downstream side of the feeding orifice 3a of hydrogen peroxide, and the feeding orifice 2a of cyclohexanone is arranged on the downstream side of the feeding orifice 4a of ammonia. Further, when the feeding orifice 4a of ammonia is set as a basis, it can be considered that the feeding orifice 2a of cyclohexanone is arranged on the downstream side of the feeding orifice 4a of ammonia, and the feeding orifice 3a of hydrogen peroxide is arranged on the downstream side of the feeding orifice 2a of cyclohexanone.

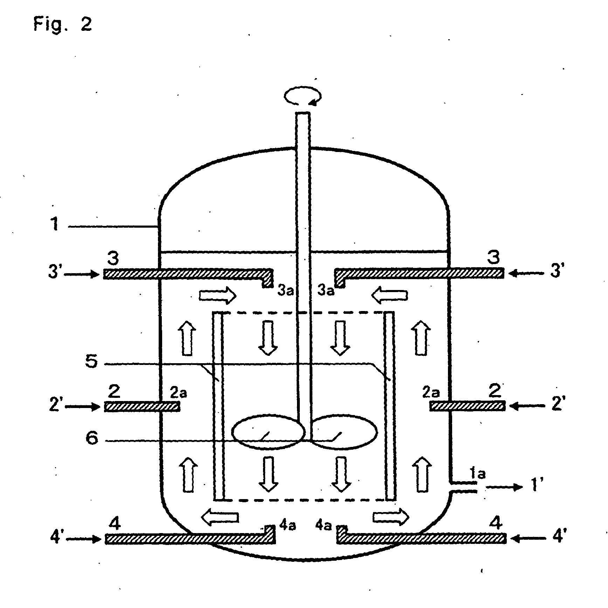

Fig. 2 schematically shows a perpendicular cross sectional view of another example of the reaction apparatus useful in the continuous ammoximation reaction process according to the present invention. In the shown example, a stirred vessel type reactor 1 is used in which a columnar separating wall 5 is provided as a bulkhead which surrounds stirring blades (or stirring impeller) 6 in order to form the circulating flow of the reaction mixture in the reactor indicated by white arrow marks. Namely, by rotating the stirring blades 6, a down flow of the reaction mixture is formed in the inside of the separating wall 5 (namely, in the central portion of the reactor 1 with respect to the horizontal cross section of the reactor), and also an up flow of the reaction mixture is formed in the outside of the separating wall 5 (namely, in the peripheral portion of the reactor 1 with respect to the horizontal cross section of the reactor). Accordingly, as a whole, the circulating flow of the reaction mixture is formed which are formed of such down flow and such up flow. Similarly to the example shown in Fig. 1, cyclohexanone 2' and 2' is fed into the circulating flow through the feeding orifices 2a and 2a at the end portions of the feed pipes 2 and 2, hydrogen peroxide 3' and 3' is fed through the feeding orifices 3a and 3a at the end portions of the feed pipes 3 and 3 which orifices are arranged at shallower liquid depth positions on the downstream sides of the orifices 2a and 2a, and further ammonia 4' and 4' is fed through the feeding orifices 4a and 4a at the end portions of the feed pipes 4 and 4 which orifices are arranged at deeper liquid depth positions on the downstream sides of the orifices 3a and 3a. Further, an amount of the reaction mixture 1' which amount is generally the same as the total amount as those fed materials is discharged through a discharge orifice 1a which is provided at the reactor 1.

It is noted that Fig. 1 shows an example in which one feeding orifice 2a, 3a or 4a is provided as to each material while Fig. 2 shows an example in which two feeding orifices 2a, 3a or 4a are provided as to each material. However, the number of the orifice(s) is any optional one, and also for example, a sparger can be used. Further, in Fig. 1 is shown an example in which the discharge orifice 1a of the reaction mixture is provided on the downstream side of the feeding orifice 3a of hydrogen peroxide and on the upstream side of the feeding orifice 4a of ammonia. In Fig. 2 is shown an example in which the discharge orifice 1a of the reaction mixture is provided on the downstream side of the feeding orifice 4a of ammonia and on the upstream side of the feeding orifice 2a of cyclohexanone. However, the position and the number of the discharge orifice(s) are optionally selected. Further, when (a) feeding orifice(s) of a solvent and/or any other material is (are) further provided although not shown in Fig. 1 or Fig. 2, one or a plurality of the orifices may be provided at any appropriate position(s).

It is desirable that the concentration of ammonia in the liquid phase of the reaction mixture discharged from the reaction system is controlled to be 1% by weight or more in order to further improve the selectivity of cyclohexanone oxime. The conversion of cyclohexanone can be also improved by such concentration control. Such concentration of ammonia is preferably 1.5% by weight or more, and usually 10% by weight or less and preferably 5% weight or less.

The positional relationship of the feeding orifice of cyclohexanone, the feeding orifice of hydrogen peroxide and the feeding orifice of ammonia with respect to the circulating flow of the reaction mixture has been already described above. Further, as to the positional relationship of the feeding orifice of cyclohexanone, the feeding orifice of hydrogen peroxide and the feeding orifice of ammonia with respect to the liquid depth direction of the reaction mixture, namely, the vertical direction, it is preferable that as shown in Fig. 2, the feeding orifice of ammonia is provided at the lowest portion and the feeding orifice of hydrogen peroxide is provided at the uppermost portion among the feeding orifice of cyclohexanone, the feeding orifice of hydrogen peroxide and the feeding orifice of ammonia. With such a preferable positional relationship, the feeding orifice of cyclohexanone may be provided for example at an intermediate position between the feeding orifice of hydrogen peroxide and the feeding orifice of ammonia as shown in Fig. 2.

(A) known process(es) can be appropriately used for a postprocessing operation of the reaction mixture obtained. For example, as described in the patent literature 3, cyclohexanone oxime can be separated by concentrating the liquid phase of the reaction mixture so as to obtain a concentrate, then extracting cyclohexanone oxime with an organic solvent from the concentrate so as to obtain an extract liquid, and then concentrating the extract liquid again.

### EXAMPLE

An example of the present invention is explained below, but the present invention is not limited by this example. In the Example, "%" and "part" which represent a content and a used amount, respectively, are based on weight unless otherwise noticed. Further, analyses of cyclohexanone and cyclohexanone oxime were carried out by using gas chromatography, and based on the analysis results, the conversion of cyclohexanone, the selectivity of cyclohexanone oxime and the yield of cyclohexanone oxime were calculated.

### Example 1

The reaction apparatus used in the Example is as schematically shown in Fig. 2. Namely, the raw materials were continuously fed,respectively, in a ratio of 6.83 parts of cyclohexanone 2', 4.44 parts of 60% hydrogen peroxide aqueous solution 3' and 2.23 parts of ammonia 4' into the stirred vessel type reactor 1 which was made of a stainless steel (SUS 316) through feeding orifices 2a, 3a and 4a. The continuous reaction was carried out under conditions of a temperature of 85°C, a pressure of 0.35 MPa (absolute pressure) and a residence time of 1.5 hours while continuously discharging the liquid phase 1' of the reaction mixture from the discharge orifice 1a through a filter and also continuously feeding tert-butyl alcohol containing water (12% water) in a ratio of 23.6 parts. During the reaction, titanosilicate (manufactured by Polimeri Europa) was present at a concentration of 30 g/L in the reaction mixture in the reactor 1. The liquid phase 1' of the reaction mixture which was discharged after 20 hours from the initiation of the reaction was analyzed, and the conversion of cyclohexanone was 99.8%, the selectivity of cyclohexanone oxime was 99.4% and the yield of cyclohexanone oxime was 99.2%. Further, the concentration of ammonia was 2%.

## Claims

1. A process for producing cyclohexanone oxime in a continuous reaction system by preparing a reaction mixture in a reactor in which mixture a titanosilicate catalyst is dispersed and into which mixture cyclohexanone, hydrogen peroxide and ammonia are fed through a feeding orifice of cyclohexanone, a feeding orifice of hydrogen peroxide and a feeding orifice of ammonia, respectively,
wherein the reaction mixture forms a circulating flow in the reactor and the feeding orifice of hydrogen peroxide and the feeding orifice of ammonia are provided in the thus indicated order on the downstream side of said circulating flow based on the feeding orifice of cyclohexanone.

2. The process according to Claim 1, wherein the concentration of ammonia in a liquid phase of the reaction mixture is 1% by weight or more.

3. The process according to Claim 1 or 2, wherein the feeding orifice of ammonia is provided at the lowest position and the feeding orifice of hydrogen peroxide is provided at the highest position among positions of the feeding orifice of cyclohexanone, the feeding orifice of hydrogen peroxide and the feeding orifice of ammonia.

## Patentansprüche

1. Verfahren zur Herstellung von Cyclohexanonoxim in einem kontinuierlichem Reaktionssystem durch Herstellen eines Reaktionsgemisches in einem Reaktor, wobei ein Titanosilikat-Katalystor in dem Gemisch dispergiert wird und wobei Cyclohexanon, Wasserstoffperoxid und Ammoniak jeweils durch eine Einspeiseöffnung für Cyclohexanon, eine Einspeiseöffnung für Wasserstoffperoxid bzw. eine Einspeiseöffnung für Ammoniak in das Gemisch eingeleitet werden,
wobei das Reaktionsgemisch eine zirkulierende Strömung in dem Reaktor bildet und die Einspeiseöffnung für Wasserstoffperoxid und die Einspeiseöffnung für Ammoniak in der so angegebenen Reihenfolge stromabwärts in der zirkulierenden Strömung, ausgehend von der Einspeiseöffnung für Cyclohexanon, bereitgestellt werden.

2. Verfahren gemäß Anspruch 1, wobei die Konzentration von Ammoniak in einer flüssigen Phase des Reaktionsgemisches 1 Gew.-% oder mehr beträgt.

3. Verfahren gemäß Anspruch 1 oder 2, wobei die Einspeiseöffnung für Ammoniak an der niedrigsten Position und die Einspeiseöffnung für Wasserstoffperoxid an der höchsten Position unter den Positionen der Einspeiseöffnung für Cyclohexanon, der Einspeiseöffnung für Wasserstoffperoxid und der Einspeiseöffnung für Ammoniak bereitgestellt wird.

## Revendications

1. Procédé de production d'oxime de cyclohexanone dans un système réactionnel en mode continu, en préparant un mélange réactionnel dans un réacteur, mélange dans lequel on a dispersé un catalyseur à base de silicate de titane et dans lequel on fait passer de la cyclohexanone, du peroxyde d'hydrogène et de l'ammoniac, respectivement, par le biais d'un orifice d'alimentation de cyclohexanone, d'un orifice d'alimentation de peroxyde d'hydrogène et d'un orifice d'alimentation d'ammoniac, le mélange réactionnel formant un courant circulant dans le réacteur et l'orifice d'alimentation de peroxyde d'hydrogène et l'orifice d'alimentation d'ammoniac étant aménagés selon l'ordre indiqué sur le côté en aval dudit courant circulant par rapport à l'orifice d'alimentation de cyclohexanone.

2. Procédé selon la revendication 1, dans lequel la concentration d'ammoniac dans une phase liquide dudit mélange réactionnel est de 1 % en poids ou plus.

3. Procédé selon la revendication 1 ou 2, dans lequel l'orifice d'alimentation d'ammoniac est aménagé dans la position la plus basse et l'orifice d'alimentation de peroxyde d'hydrogène est aménagé dans la position la plus élevée parmi les positions de l'orifice d'alimentation de cyclohexanone, de l'orifice d'alimentation de peroxyde d'hydrogène et de l'orifice d'alimentation d'ammoniac.
